# EUROPEAN PATENT SPECIFICATION

(11) **EP 1 567 018 B1**
(45) Date of publication and mention of the grant of the patent: **01.07.2009**
(21) Application number: 02783892.9
(22) Date of filing: 18.10.2002
(51) Int. Cl.: A23K 1/00, A61K 35/74, C12R 1/225, A23L 1/03

(54) **METHOD OF IMPROVING IMMUNE FUNCTION IN MAMMALS USING LACTOBACILLUS REUTERI STRAINS**
VERFAHREN ZUR VERBESSERUNG DER IMMUNFUNKTION BEI SÄUGERN UNTER VERWENDUNG VON LACTOBACILLUS REUTERI-STÄMMEN
PROCEDE PERMETTANT DE RENFORCER LA FONCTION IMMUNITAIRE CHEZ LES MAMMIFERES AU MOYEN DE SOUCHES DE LACTOBACILLUS REUTERI

(43) Date of publication of application: 31.08.2005
(73) Proprietor: BIOGAIA AB, 103 64 Stockholm (SE)
(72) Inventor: Kang, Ho-Jin, Incheon (KR); Kwon, Ik-Boo, Dongjak-ku, Seoul (KR); CONNOLLY, Eamonn, S-181 30 Lidingö (SE); MÖLLSTAM, Bo, S-443 31 Lerum (SE); LEE, Dong-Seog, Goyang, Keonggi-do (KR)
(74) Representative: Fagerlin, Heléne
(86) International application number: PCT/SE2002/001903
(87) International publication number: WO 2004/034808

(56) References cited:
- WO-A1-94/00139
- WO-A1-99/17788
- CASAS IVAN A. ET AL.: 'Lactobacillus reuteri: An effective probiotic for poultry and other animals' FOOD SCIENCE & TECHNOLOGY vol. 85, 1998, pages 475 - 518, XP002988456
- WATERS W. R. ET AL.: 'Effects of lactobacillus reuteri on cryptosporidium parvum infection of gnotobiotic TCR-alpha-deficient mice' EUKARYOT MICROBIOL., [Online] vol. 46, no. 5, September 1999, page 60S, XP008051110 Retrieved from the Internet: <URL:http://www.nps.ars.usda.gov/publicatio ns/publications.htm?SEQ-NO-115=106105>

## Description

### BACKGROUND OF THE INVENTION

### Field of the Invention

This invention relates to the use of *Lactobacillus reuteri* strain ATCC55730 as immune enhancing agent.

### Description of the Related Art

Probiotics containing a wide variety of different gastrointestinal microorganisms have been formulated, primarily because of the increase in antibiotic-resistant pathogens. Strains of a wide variety of *Lactobacillus* species, including *L. reuteri* have been used in probiotic formulations. *Lactobacillus reuteri* is one of the naturally occurring inhabitants of the gastrointestinal tract of animals, and is routinely found in the intestines of healthy animals. It is known to have antibacterial activity. See, for example U.S. Patent Nos. 5,439,678, 5,458,875, 5,534,253, 5,837,238, and 5,849,289. When *L. reuteri* cells are grown under anaerobic conditions in the presence of glycerol, they produce the antimicrobial substance known as reuterin (β-hydroxy-propionaldehyde).

Immunomodulating activity has also been associated with *L. reuteri.* See, for example "Biotherapeutic effects of probiotic bacteria on candidiasis in immunodeficient mice" by Wagner RD, et al, Infect Immune 1997 Oct 65:4165-72; however, differences in efficacy exists between strains and methods are needed to select the most effective strains, for example the method selecting strains recruiting CD4+ cells, provided in this invention.

WO-A-9 400 139 discloses a method of stimulating the immune system of poultry.

Furthermore, although *L. reuteri* is known to be used as a generally beneficial probiotic, previous work has only to some extent realized the importance of utilizing best *Lactobacillus* strains that neutralize toxins produced by these pathogens already present in the gastrointestinal tract. See, for example, "Removal of common Fusarium toxins in vitro by strains of Lactobacillus and Propionibacterium" by. El-Nezami HS, et al, Food Addit Contam 2002 Jul 19:680-6. Such toxin neutralization, including binding, is not only as reported important for ameliorating the direct effects caused by these toxin producing pathogens but also in reducing the general burden on the immune-system, according to this invention.

There are many different causes of gastrointestinal problems caused by pathogenic microorganisms. For example, *Helicobacteri pylori* causes gastric and duodenal ulcers, gastric cancer, and gastric mucosa-associated lymphoid tissue lymphoma. Certain pathogenic *Escherichia coli* strains produce toxins such as the vero toxin (VT) produced by *E. coli* O157:H7, against which antibiotics are less and less effective.

It is therefore an object of the invention to provide a method of improving immune-function in mammals using *Lactobacillus reuteri* strains detected to be effective in both CD4+ cell recruitment and in toxin neutralization, and to provide methods of selecting such immune-improving *L. reuteri* strains, and to provide products containing such strains. It is a further object of this invention to provide a method of utilizing culture supernatants of strains of *L. reuteri* effective in reducing the immune burden of these toxins.

Other objects and advantages will be more fully apparent from the following disclosure and appended claims.

### SUMMARY OF THE INVENTION

The invention herein is related to the use of *Lactobacillus reuteri* strain ATCC55730 as immune enhancing agent, and methods of improving immune-function in mammals using *Lactobacillus reuteri* strains in products containing cells of such strains, This strain exhibits good toxin binding and neutralizing; and exhibits good CD4+ recruitment. Other objects and features of the inventions will be more fully apparent from the following disclosure and appended claims.

### BRIEF DESCRIPTION OF THE DRAWINGS

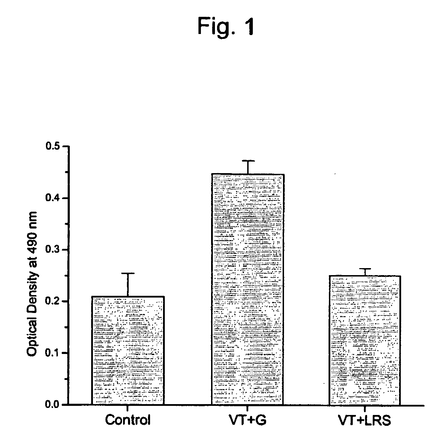

Figure 1. Confirmation of inhibitory ability against the binding of vero cytotoxin(VT) and Gb₃ receptor in a culture supernatant of *L. reuteri* through competitive ELISA. Each reacted as follows, on plates coated with Gb₃, followed by performing ELISA using mAb against VT.

Control: VT + tryptic soy broth(TSB)
VT + G: VT + 250 mM glycerol solution
VT + LRS: VT + a culture supernatant of *L. reuteri* incubated in 250 mM glycerol solution

### DETAILED DESCRIPTION OF THE INVENTION AND PREFERRED EMBODIMENTS THEREOF

The present invention relates to the use of *Lactobacillus reuteri* strain ATCC55730 that exhibits good toxin binding and neutralizing effect; and exhibits good CD4+ cell recruitment for the production of a composition for improving immune-function in mammals. It also relates to the use of such a *Lactobacillus reuteri* strain in products and a method of improving immune-function in mammals using such a Lactobacillus reuteri strain.

Cytokines are immune system proteins that are biological response modifiers. They coordinate antibody and T cell immune system interactions, and amplify immune reactivity (10). Cytokines include monokines synthesized by macrophages and lymphokines produced by activated T lymphocytes and natural killer (NK) cells. The CD4+ subset in both human and mice is based on cytokine production and effector functions. Th1 cells synthesize interferon- gamma (INF-γ), IL-2 and tumor necrosis factor (TNF). They are mainly responsible for cellular immunity against intracellular microorganisms and for delayed-type-hypersensitivity reactions. They affect Immunoglobulin G 2a (IgG2a) synthesis and antibody dependent cell mediated cytotoxicity. The INF-γ they produce activates macrophages and consequently phagocytosis. Th2 cells synthesize interleukin-4 (IL-4), interleukin-5 (IL-5), interleukin-6 (IL-6), interleukin-9 (IL-9), interleukin-10 (IL-10), and interleukin-13 (IL-13). They induce IgE and Ig G1 antibody responses, and mucosal immunity by synthesis of mast cells and eosinophil growth and differentiation factors, and facilitation of IgA synthesis.

Included in the invention are methods with various example steps confirming: administration of the *L.reuteri* strain, analysis of the strain and efficacy of the used strain in both CD4+ cell recruitment and toxin neutralization. Data indicate that for example a tablet formulation containing *L. reuteri* gives similar gastrointestinal colonization levels as with direct administration of cell cultures. The *L. reuteri* colonization is related to ingestion of *L. reuteri,* and the washout period (the time it takes for *L. reuteri* levels to drop to pre-ingestion levels) is at least 28 days after administration of the tablet, showing that the invention herein is applicable for *L. reuteri* cell cultures as well as products formulated to contain such *L.reuteri* cultures.

The invention preferably relates to the use of products comprising *L.reuteri* strains as a probioticum for prophylactic improvement of the immune-function in mammals. Such products may be various foodstuffs such as a dietary supplement, confectionery or tablets containing cells of the selected strain.

The *L.reuteri* strains according to the invention may also be used for the preparation of a drug for treatment of various microorganisms that produce toxin, such as Echericia coli. Enterohemorrhagic E. Coli and VT toxins may thus be treated.

According to the invention both cells of *L.reuteri* strains and a culture supernatant thereof may be used for the production of a prophylactic, probiotic type and pharmaceutical composition.

According to the invention any The *L.reuteri* strain exhibiting good CD4+ cell recruitment and /or good toxin binding may be used. That CD4+ cells are present may be tested using antibodies against CD4 e.g. with immunohistochemical (such as in example 5) or immunoflourescent methods. Toxin binding may be confirmed by bringing *L.reuteri* cells or supernatant in contact with toxin and test for the difference in available toxin e.g. as is done in example 1.

The probiotic, prophylactic and pharmaceutical products according to the invention may comprise additives and excipients acceptable for nutritious or pharmaceutical use.

The features of the present invention will be more clearly understood by reference to the following examples, which are not to be construed as limiting the invention.

### Example 1. Vero toxin study

Strains of three lactic acid bacteria, *L. reuteri* ATCC 55730, *L. bulgaricus, strain LBS12,* (*CHR, Horsholm, Denmark*) , *and L. casei,* strain 0 1, (CHR, Horsholm, Denmark), were employed in this experiment. *L. reuteri* was incubated in an aerotropic fixing condition at 37°C for 24-48 hours after inoculating in MRS broth (plus 20 mM glucose). In some cases, this initial incubation was followed by centrifugation at 2,500 rpm for 30 minutes, washing with phosphate buffered saline (PBS) twice to remove medium components, suspension in 250 mM glycerol solution, followed by incubation in an aerotropic fixing condition at 37°C for 6 hours. *L. bulgaricus* and *L. casei* were incubated on MRS plus 20 mM glucose (without glycerol) in an aerotropic fixing condition at 37°C for 24-48 hours. Each test lactic acid bacterium was employed following adjusting to 2g/30 ml (dry weight), centrifuging at 2,500 rpm for 30 minutes after incubation, retrieving the supernatant, adjusting the pH to 7.0 with NaOH to inoculate vero cells (see below), and filtering through a 2.0 µm filter. Glycerol solution and MRS broth adjusted to pH 7.0 served as a control, following filtering through a 2.0 µm filter.

Vero cells (African Green Monkey kidney cells, ATCC - CCL81) were incubated in a minimum essential medium (MEM, Sigma) supplemented with 10% fetal bovine serum (FBS, Sigma) at 25 g/ml gentamycin (Sigma) at 37°C in a 10% CO₂ incubator for 48 hours, and were used after confirming monolayer formation.

*Escherichia coli* 0157:H7 (ATCC 43894), which secretes both VT1 and VT2, was employed, following inoculating to tryptic soy broth (TSB), incubating for 24 hours while stirring in a shaking incubator at 37°C, centrifuging at 2,500 rpm for 30 minutes, and filtering the culture supernatant.

500 Vero cells (2 x 10⁵ cells/ml) were inoculated to a 96-well plate and incubated at 37°C in a 10% CO₂ incubator for 48 hours to confirm formation of a monolayer, followed by carrying out the experiment using the following treatments: A: VT only (positive control); B: TSB (*Escherichia coli* 0157:H7 culture medium); C: MRS broth (test lactic acid bacteria); D: glycerol solution (*L. reuteri* culture medium); E: VT + MRS broth; F: VT + glycerol solution; G: VT + glycerol solution culture supernatant of *L. reuteri;* H: VT + MRS broth culture supernatant of *L. bulgaricus*; I: VT + culture supernatant of *L. bulgaricus*; and J: VT + culture supernatant of *L. casei.* Treatments B, C and D are to determine whether each culture fluid itself causes cytotoxicity to Vero cells; and E and F are to determine whether culture media of test lactic acid bacteria have neutralizing capability against VT in themselves. Each culture supernatant of test lactic acid bacteria in G, H, I and J was subject to 2x serial dilution, after each of the diluted culture supernatants of test lactic acid bacteria and VT were combined by 400 ml + 100 ml; 300 ml + 200 ml; 200 ml + 300 ml; and 100 ml + 400 ml, respectively, and then incubated at 37°C in a 10% CO₂ incubator for 18 hours to determine whether a cytopathic effect (CPE) appeared.

300 mM neutralized semicarbazide hydrochloride (Sigma), which inhibits reuterin production, was added to a culture fluid of *L. reuteri* in 250 mM glycerol solution to restrain reuterin production, and the supernatant collected as above. 100 µl Vero cells were inoculated to a 96-well plate and incubated at 37°C in a 10% CO₂ incubator for 24 hours to examine whether monolayers formed, following by performing the following treatments: A: VT only; B: VT + 25 mM glycerol solution; C: VT + glycerol solution culture supernatant of *L. reuteri;* and D: VT + glycerol solution culture supernatant of *L. reuteri* incubated after treatment with reuterin inhibitor (see above). Each culture fluid of *L. reuteri* treated with or without glycerol solution and reuterin inhibitor in B, C and D, was combined with VT by 20 + 80; 30 + 70; 40 + 60; 50 + 50; 60 + 40; 70 + 30; and 80 + 20 µl respectively, and then incubated at 37°C in a 10% CO₂ incubator for 18 hours to examine whether a cyptopathic effect appeared. After microscopic observation, culture fluids were dyed with crystal violet and their O.D. (optical density) values were read at 490 nm.

96-well plates were coated with Gb3 (globotriaosylceramide) (Sigma) blocked with 5% bovine serum albumin (BSA) and reacted with *L. reuteri* culture supernatant that was incubated in either VT or VT + 250 mM glycerol solution. Afterward, a monoclonal antibody (mAb) against VT was used as a primary antibody, to which horseradish peroxidase (HRP)-conjugated goat anti-mouse IgG was added, and, following development of O-phenylene diamine, its optical density (O.D.) was read at 490 nm through an ELISA reader. This experiment confirmed the presence of any material inhibiting the interaction between *L. reuteri* and the Gb₃ receptor.

From the results of interaction between VT and 250 mM glycerol solution and between VT and a culture supernatant of *L. reuteri* incubated in glycerol solution after coating with Gb₃, it was ascertained that a combination of VT and a culture supernatant of *L. reuteri* yielded a low O.D. value at significant levels compared with that of VT only, as in Figure 1. This means that the presence of a material that inhibited binding of VT and the Gb₃ receptor in a culture supernatant of *L. reuteri* could be detected indirectly.

96-well plates were coated with *L. reuteri* culture supernatant which was incubated in VT/VT + 250 mM glycerol solution, blocked with 3% BSA and reacted with VT. Afterward, a monoclonal antibody against VT was used as a primary antibody, to which horseradish peroxidase (HRP)-conjugat4ed goat anti-mouse IgG (H+L) was added, and, following development of O-phenylene daimine, its optical density (O.D.) was read at 490 nm through an ELISA reader. This experiment confirmed the presence of an interactive material with VT in the *L. reuteri* supernatant

### Example 2. Investigation of neutralizing effect of lactic acid bacteria on Vero cytotoxin (VT) I and II secreted by E. coli O157:H7

When TSB, MRS broth and glycerol solution were added to Vero cells, a cytopathic effect was not seen. In addition, when both VT and MRS broth/glycerol solution were added to Vero cells, CPE was observed in Vero cells, which proves that the culture fluids themselves lacked a neutralizing capability against VT.

When each culture supernatant of test lactic acid bacteria was subjected to adjusting to pH 7.0, filtering and combining with VT, the results shown in Table 1 were found. For *L. bulgaricus* and *L. casei,* CPE appeared in the entire range of concentrations, while for *L. reuteri,* CPE did not appear in many of the glycerol supernatants, except with the ratio of test lactic acid bacteria to VT of 4:1, where there was much less CPE. Thus, there was a discernible neutralizing capability against VT of the culture supernatant incubated in 250 mM glycerol solution. For incubation in MRS broth (+20 mM glucose), CPE appeared at all concentrations.

**Table 1. Comparison of inhibitory ability against cytopathic effect(CPE) of vero cells by vero cytotoxin(VT) in culture supernatants of L. reuteri, L. bulgaricus and L. casei**

| Lactic acid bacteria | lactic acid bacteria culture supernatants(µl) | VT(µl) | serial dilution(x 2) of lactic acid bacteria culture supernatants | | | | |
|---|---|---|---|---|---|---|---|
| | | | 0 | 1 | 2 | 3 | 4 |
| *L. reuteri* (G)* | 400 | 100 | - | - | - | +^{a} | +^{a} |
| | 300 | 200 | - | - | +^{a} | + | + |
| | 200 | 300 | - | +^{a} | + | + | + |
| | 100 | 400 | +^{a} | +^{a} | + | + | + |
| *L. reuteri* (MRS)** | 400 | 100 | +^{a} | + | + | + | + |
| | 300 | 200 | + | + | + | + | + |
| | 200 | 300 | + | + | + | + | + |
| | 100 | 400 | + | + | + | + | + |
| *L. bulgaricus* | 400 | 100 | + | + | + | + | + |
| | 300 | 200 | + | + | + | + | + |
| | 200 | 300 | + | + | + | + | + |
| | 100 | 400 | + | + | + | + | + |
| *L. casei* | 400 | 100 | + | + | + | + | + |
| | 300 | 200 | + | + | + | + | + |
| | 200 | 300 | + | + | + | + | + |
| | 100 | 400 | + | + | + | + | + |

| | | | | | | | |
|---|---|---|---|---|---|---|---|
| *: The culture supernatant obtained following incubating *L. reuteri* in 250 mM glycerol solution **: The culture supernatant obtained following incubating *L. reuteri* in MRS broth(adding 20 mM glucose) -: No CPE(cytopathic effects) +: CPE a: Mild CPE | | | | | | | |

The results of the competitive ELISA and the binding assay between VT and *L. reuteri* culture supernatant were subjected to Student's t test using a Microcal Origin 6.1 (Microcal Software, Inc., Boston, MA) program.

### Example 3. Administration of L. reuteri to subjects

In this example subjects were given two chewing tablets twice per day, each tablet containing 1 x 10⁸ CFU (colony-forming units) of *L. reuteri* (SD2112: ATCC 55730) to give a total daily dose of 4 x 10⁸ CFU *L. reuteri.* All other excipients used in the tablets were well-known and complied with international pharmacopoeias. The study was performed in two parts: a gastroscopy session with investigation of the upper gastrointestinal tract, and an ileoscopy session with investigation of the distal small bowel (details below). The exclusion criteria were: antibiotics taken two weeks before and during the study; probiotics taken three weeks before and during the study, ongoing treatment with gastro intestinal related drugs, and severe organic disease with need of regular treatment (e.g., cancer). The protocol for patient treatment was approved by the Danish Ethical Committee and was in accordance with the declaration of Helsinki. The study was done in Denmark.

The Wilcoxon signed-rank test was used to compare symptoms, blood test values, stool content *of L. reuteri,* and histological differences before and after intake of *L. reuteri.* P < 0.05 was considered significant.

All subjects completed the study. In the gastroscopy session, ten healthy volunteers, aged greater than 18 years, with normal eating habits, were studied. A gastroduodenoscopy was performed following an overnight fast on day 0, before intake of *L. reuteri,* and on day 28, at the end of the study. Biopsies were taken from the corpus and the antrum of the stomach and from the third part of the duodenum, both for culture *of L. reuteri* and for histological examination. The average size of the biopsy was 33 mg. The biopsies were immediately placed in PBS (2:1 vol/wt of sample), stored on ice and transported to Biogaia AB Laboratories in Lund, Sweden for analysis of *Lactobacillus reuteri* counts. The time from the isolation of tissue to analysis was within 36 hours.

In the ileoscopy session, nine subjects with ileostomy following colectomy for ulcerative colitis (3 subjects) or Crohn's disease (6 subjects) were studied. The small bowel was without signs of inflammation in all subjects. An ileoscopy was performed on day 0 and day 28. Biopsies were taken as above within the distal 20 cm of the small bowel for culture of *L. reuteri* as well as other lactobacilli and for histological examination.

### Example 4. Analyses for Microorganism

Stool samples were collected from each subject before intake of L. reuteri (day 0) and at the end of the study (day 28). Feces in ileoscopy subjects (explained below) were taken from the stoma. Seven volunteers from the gastroscopy session and four volunteers from the ileoscopy session collected stool samples at day 42 (14 days after cessation of the L. reuteri intake). The samples (no les than 5 g) were collected into a sterile container and placed immediately in a refrigerator. Within 24 hours, 20 ml (1:5, wt/vol) of 0.1% peptone water was added. The sample was homogenized and aliquots were dispensed into cryo-vials before freezing immediately at -70°C. The samples were shipped, frozen on dry ice, to Biogaia AB laboratory for analysis of total Lactobacillus and L. reuteri. There, the samples were thawed, diluted and plated on MRS-3 agar containing vancomycin (50 mg/l) for the L. reuteri and LBS agar plates (KEBOLAB AB, Lund, Sweden) ) for the total Lactobacillus count. MRS-3 is a modified MRS agar (KEBOLAB AB, Lund, Sweden) containing 2% sodium acetate (wt/vol). LBS agar is prepared as recommended by the manufacturer by adding 1.32 ml glacial acetic acid per liter. Agar plates were incubated anaerobically using BBL Gas packs in anaerobic jars) at 37°C for 48 hours. DNA from selected isolates from the study (see below) were analyzed by PCR using a Bacterial Barcodes repPROTM DNA Fingerprinting kit (Bacterial BarCodes, Inc., Houston, TX), and the fingerprints were analyzed using Bionumerics software (Applied Maths BVBA, Sint-Martens-Latem, Belgium).

At the start of the study and before administration of the Study Product, none of the gastroscopy session subjects had detectable *L. reuteri* in the feces (Table 2). After 28 days *of L. reuteri* intake, all of them had *L. reuteri* in the feces in amounts ranging from 1.0 x 10² to 3.5 x 10⁵ CFU/g (colony-forming units per gram fecal material), with a mean of 4.0 x 10⁴ CFU/g, indicating a significant increase in live *L. reuteri* in the feces due to administration of the Study Product. Five subjects elected to deliver fecal samples on days 42 and 56 (i.e., up to 28 days after administration of the Study Product). In these subjects, the *L. reuteri* persisted in the feces four weeks after intake (Table 2).

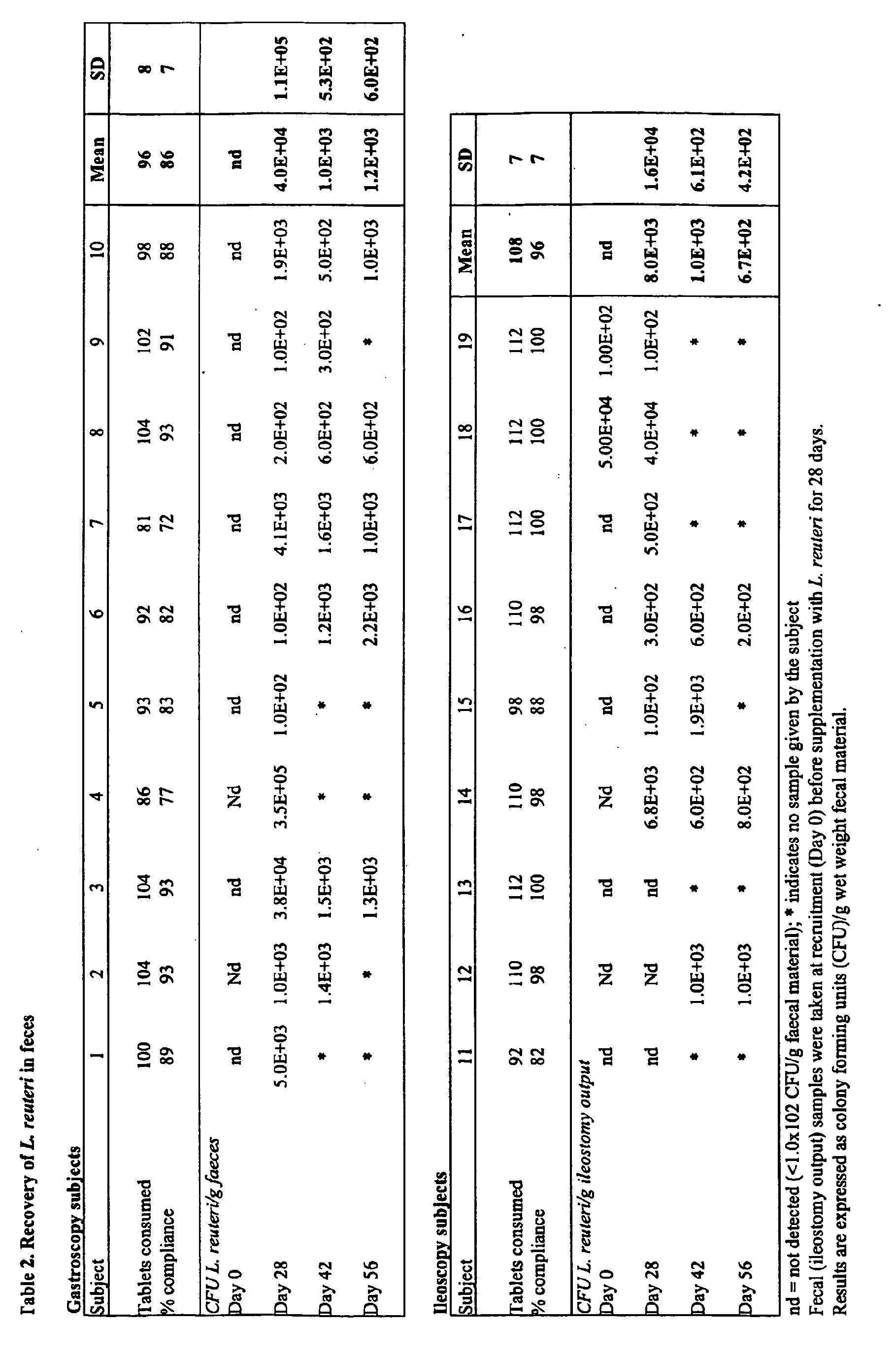

### Example 5. Histology

To evaluate any local immunological response, the changes in amount of B-lymphocytes, T-lymphocytes and macrophages were determined. Baseline biopsies and day-28 biopsies were formalin-fixed and imbedded in paraffin. Subsequently, 4 µm sections were cut and stained histochemically using standard techniques (Hematoxylineosin, van Gieson, PeriodicAcidSciff-Alcain and PeriodicAcidSciff-diastase) and immunohistochemically. The primary antibodies, obtained from DAKO, Glostrup, Denmark, were to: CD20 (B-lymphocytes), CD3, CD4+, CD8 (T-lymphocytes), CD68 (histiocytes), *Helicobacter,* and Ki-67 (proliferation marker). The immunohistochemical staining was performed on the DAKO TechMate^{™} 500 immunostainer to obtain uniform staining.

A pathologist evaluated the biopsies. On the basis of histochemical staining the tissue damage was graded (1-10) according to Madsen et al. (Gastroenterol. 1999; 116:1107-1114). This grade represents the numerical sum of four criteria: ulceration, epithelial hyperplasis, the amount of mononuclear cells, and neutrophil granulocytes in the lamina propria. On the basis of the immunohistochemical staining, the number of CD20, CD3, CD4+, CD8 and CD68 positive cells in lamina propria were evaluated semi-quantitatively. Three months later, the baseline and day-28 biopsies were blinded and reevaluated by the same pathologist. The overall correlation between positive stained cells of the baseline and the day-28 biopsies were calculated in each evaluation and compared.

Predominantly single B-lymphocyte cells were found in the stomach (corpus and antrum) of two subjects at day 0 and two subjects on day 28. One subject had dispersed cells in the stomach (antrum) on day 28. Four subjects had single cells in the duodenum at day 0 and 8 subjects had predominantly single cells in the duodenum at day 28. Eight subjects had predominantly single cells of CD3, CD4+ and CD8 (T-lymphocytes) in the stomach (corpus and antrum) at day 0 and nine subjects had predominantly single cells in the ventricle (corpus and antrum) at day 28 (Table 3). Dispersed cells were found in the duodenum in 8 subjects at day 0 and predominantly dispersed T-lymphocytes were found in the duodenum of 7 subjects at day 28.

Predominantly single histiocyte cells were found in the stomach (corpus and antrum) in nine subjects at day 0 and in 5 subjects at day 28. Predominantly dispersed cells were found in the duodenum of 6 subjects at day 0 and day 28 (not the same 6 subjects).

There was a statistically significant fall in the number of histiocytes (CD68) in the biopsies from the corpus (p=0.025) and antrum (p=0.046), and a significantly increased number of B-cells (CD20) in the duodenum after subjects had received *L. reuteri* (p=0.046); Table 3). No significant changes were found in the amount of T-lymphocytes due to the intake of *L. reuteri.* Concordance between the two investigations (first, not-blinded, and second, blinded analysis of histological samples) was 76% (Table 3). For the histiocytes (CD68) cells of the corpus and antrum, the concordance was only 40% and 65%, respectively. Concordance of the duodenal B-cells (CD20) was 60%. Thus, there was some uncertainty in these findings. Concordance in the CD3, CD4+, and CD8 analyses was much higher (up to 90%; Table 3).

In the ileoscopy session, all biopsies were histologically normal and *Helicobacter* negative. Ki-positive cells were normal in all biopsies. Predominantly single B-lymphocyte cells were found in 6 subjects and dispersed cells in 2 subjects at day 0. Single cells were observed in all subjects at day 28. Predominantly dispersed CD3 cells (B-lymphocytes) were found in 7 subjects on both day 0 and day 28. Predominantly dispersed CD4+ cells were found in 7 subjects on day 0 and predominantly adjoining groups of cells in 7 subjects on day 28. Predominantly dispersed CD8 cells were found in 7 subjects on day 0 and in 9 subjects on day 28 (Table 3). Predominantly dispersed histiocyte cells were found in 7 subjects on both day 0 and day 28.

There was a significantly higher amount of CD4+ lymphocytes after supplementation with *L. reuteri* for 28 days (p=0.046; Table 3). No significant changes were found in the amount of B-lymphocytes or histiocytes due to the administration of *L. reuteri.* Concordance between the two investigations was generally high for all cells types examined and for the CD4+ findings it was 78% indicating good reliability in these data (Table 3). All *Helicobacter* stainings were negative.

**Table 3. Histological evaluation of ileal biopsies from subjects in the ileoscopy session**

| Subject | **Day 0** Histology | B-cells | | T-cells | | Histiocyte | **Day 28** Histology | B-cells | | T-cells | | Histiocyte |
|---|---|---|---|---|---|---|---|---|---|---|---|---|
| | score | CD 20 | CD 3 | CD4 | CD8 | CD68 | Score | CD 20 | CD 3 | CD4 | CD8 | CD 68 |
| 11 | 0 | X | XX | XX | XX | XX | 0 | X | X | XXX | XX | XX |
| 12 | 0 | XX | XXX | XXX | XX | XX | 0 | X | XX | XXX | XX | XX |
| 13 | 0 | - | XX | XX | XX | XX | 0 | X | XX | XXX | XX | X |
| 14 | 0 | X | XX | XX | XX | XX | 0 | X | X | XXX | XX | XX |
| 15 | 0 | XX | XXX | XX | XX | XX | 0 | X | XX | XXX | XX | X |
| 16 | 0 | X | XX | XX | XX | XX | 0 | X | XX | XXX | XX | XX |
| 17 | 0 | X | XX | XX | X | XX | 0 | X | XX | XX | XX | XX |
| 18 | 0 | X | XX | XX | X | X | 0 | X | XX | XX | XX | XX |
| 19 | 0 | X | XX | XXX | XX | X | 0 | X | XX | XXX | XX | XX |

| | | | | | | | | | | | | |
|---|---|---|---|---|---|---|---|---|---|---|---|---|
| Biopsies were taken within the distal 20cm of the samll bowel and were fixed and sectioned for histological Examination using immunohistochemical staining of specific cell types (see text). "-" = no cells detected; "X "= single cells; "XX" = dispersed cells or aggregations of cells; "XXX"= several adjoining groups of cells. Histology score (1 to 10) grading the degree of tissue damage. Statistical analysis: Wilcoxon Signed Rank Test. The samples were given a nominal score -, X, XX and XXX were set to 1,2,3 & 4 respectively for statistical analysis. | | | | | | | | | | | | |

### Example 6. Biochemistry of blood

Blood samples were taken on day 0 and day 28, and were analyzed for hemoglobin, hematocrit, thrombocytes, luecocytes, C-reactive protein, potassium, sodium, creatinine, b-urea, p-glucose, cholesterol, HDL (high density lipoproteins), LDL (low density lipoproteins), VLDL (very low density lipoproteins), tricgycerides, total bilirubin, urate, ALAT, alkaline phosphatase and lactate.

Most blood tests were normal, both before and after intake of *L. reuteri.* There were a few outliers in the blood variables, but no clinically significant abnormalities were found and no systematic changes were observed following the treatment.

### Example 7. DNA Fingerprinting

DNA fingerprinting analysis was performed on selected *L. reuteri* isolates from the study. Thus, fecal isolates from three subjects who had consumed *L. reuteri* for 28 days were taken as well as one isolate from a duodenal biopsy and one isolate from an ileal biopsy both taken on day 0 before *L. reuteri* administration. All isolates were found to have a 98% genetic similarity to each other. All of these isolates showed a 97% similarity to SD2112, the strain incorporated into the tablets.

### Example 8: Formulation of product to improve immune-function in humans

In this example is *L. reuteri* SD2112, ATCC 55730 selected, using the methods above for toxin neutralization and CD4+ cell recruitment, in order to add to a standard yogurt. The *L. reuteri* strain is grown and lyophilized, using standard methods for growing *Lactobacillus* in the dairy industry. This culture are then added to previously fermented milk, using traditional yogurt cultures, at a level of 10E+7 CFU/gram of yogurt, and the yogurt is used by humans as a way to improve their immune function.

While the invention has been described with reference to specific embodiments, it will be appreciated that numerous variations, modifications, and embodiments are possible, and accordingly, all such variations, modifications, and embodiments are to be regarded as being within the spirit and scope of the invention.

## Claims

1. Use of *Lactobacillus reuteri* ATCC55730 that
a. Exhibit good toxin binding and neutralizing effect;
b. Exhibit good CD4+ cell recruitment
for the producing of a composition for improving immune-function in mammals.

2. The use according to claim 1, wherein a culture supernatant of *L. reuteri* ATCC 55730 is used for neutralizing bacterial Toxins.

3. The use according to claim 1, wherein the product is formulated as a food containing cells of the selected strain.

4. The use according to claim 3, wherein the product is formulated as a tablet containing cells of the selected strain.

5. The use according to claim 3, wherein the product is formulated as a dietary supplement containing cells of the selected strain.

6. The use according to claim 3, wherein the product is formulated as a confectionery containing cells of the selected strain.

7. The use according to claim 3, wherein the product is formulated as a drug containing cells of the selected strain.

## Patentansprüche

1. Verwendung von *Lactobacillus reuteri* ATCC 55730, der
a) eine gute Toxinbindewirkung und eine gute Toxinneutralisierungswirkung aufweist;
b) eine gute CD4+-Zellenrekrutierung aufweist,
zur Produktion einer Zusammensetzung zur Verbesserung der Immunfunktion von Säugetieren.

2. Verwendung nach Anspruch 1, wobei ein Kulturüberstand von *L. reuteri* ATCC 55730 zum Neutralisieren von bakteriellen Toxinen verwendet wird.

3. Verwendung nach Anspruch 1, wobei das Produkt als Nahrungsmittel formuliert ist, das Zellen des gewählten Stammes enthält.

4. Verwendung nach Anspruch 3, wobei das Produkt als Tablette formuliert ist, die Zellen des gewählten Stammes enthält.

5. Verwendung nach Anspruch 3, wobei das Produkt als Nahrungsergänzungsmittel formuliert ist, das Zellen des gewählten Stammes enthält.

6. Verwendung nach Anspruch 3, wobei das Produkt als Süßware formuliert ist, die Zellen des gewählten Stammes enthält.

7. Verwendung nach Anspruch 3, wobei das Produkt als Arzneimittel formuliert ist, das Zellen des gewählten Stammes enthält.

## Revendications

1. Utilisation du *Lactobacillus reuteri* ATCC55730 qui
a. présente un bien liaison avec toxin et une bien effet de neutralisation;
b. présente une bien recrutement des cellules CD4+
pour le production d'une composition pour améliorer la fonction immunologique chez des mammifères.

2. L'utilisation selon la revendication 1, dans laquelle un supernageant d'une culture de *L.reuteri* ATCC 55730 est utilisé pour la neutralisation des toxines bacterielles.

3. L'utilisation selon la revendication 1, dans laquelle le produit est formulé comme un aliment contenant des cellules de la souche selectionnée.

4. L'utilisation selon la revendication 3, dans laquelle le produit est formulé comme une comprimé contenant des cellules de la souche selectionnée.

5. L'utilisation selon la revendication 3, dans laquelle le produit est formulé comme un supplement alimentaire contenant des cellules de la souche selectionnée.

6. L'utilisation selon la revendication 3, dans laquelle le produit est formulé comme une confiserie contenant des cellules de la souche selectionnée.

7. L'utilisation selon la revendication 3, dans laquelle le produit est formulé comme un médicament contenant des cellules de la souche selectionnée.
